# EUROPÄISCHE PATENTSCHRIFT

(11) **EP 0 983 756 B1**
(45) Veröffentlichungstag und Bekanntmachung des Hinweises auf die Patenterteilung: **02.04.2003**
(21) Anmeldenummer: 99114097.1
(22) Anmeldetag: 20.07.1999
(51) Int. Cl.: A61F 5/01

(54) **Sichelfuss-Korrekturschiene**
Anti-adductus correction splint
Atelle corrigeante d'anti-adductus

(30) Priorität: 20.07.1998 DE 29812781 U
(43) Veröffentlichungstag der Anmeldung: 08.03.2000
(73) Patentinhaber: Oehler, Claus, Dr., 86150 Augsburg (DE)
(72) Erfinder: Dr Oehler Claus, 86150 Augsburg (DE)
(74) Vertreter: Ernicke, Hans-Dieter, Dipl.-Ing.

(56) Entgegenhaltungen:
- EP-A- 0 027 762
- EP-A- 0 533 969
- DE-A- 3 543 642
- DE-U- 9 418 854
- DE-U- 29 812 781

## Beschreibung

Die Erfindung betrifft eine Sichelfuß-Korrekturschiene mit den Merkmalen im Oberbegriff des Hauptanspruches.

Eine solche Sichelfuß-Korrekturschiene ist aus der DE-A-35 43 642 bekannt. Sie besitzt zwei Schalenteile für Ferse und Vorderfuß, die durch seitlich angeordnete Beschläge mit einem Gelenk drehbar miteinander verbunden sind. Das Gelenk befindet sich auf der lateralen Fußseite. Seine Winkelstellung kann durch eine Klemmschraube eingestellt und arretiert werden. Dafür ist ein Spezialwerkzeug erforderlich. Außerdem besteht durch die Arretierung im Gelenk das Problem, daß die gewählte Winkelstellung der beiden Fußfassungen bei der Arretierung des Gelenkes ungewollt verändert wird.

Die EP-A 0 533 969 zeigt eine andere Sichelfuß-Korrekturschiene mit zwei Fußfassungen, die im Sohlenbereich unter dem Fuß durch Beschläge und ein Gelenk drehbar miteinander verbunden sind. Das Gelenk ist lateral angeordnet, wobei die Feder als Druckfeder oder als Torsionsfeder ausgebildet ist und auf der medialen Seite wirken soll. Durch die Feder wird zwar eine permanente Stellkraft auf die Fußfassungen ausgeübt, die jedoch nicht in ihrer Größe einstellbar ist. Durch die Anordnung von Beschlag und Feder unter der Sohle der Fußfassungen kann der Patient weder gehen noch stehen.

Die EP-B-0 027 762 zeigt einen Stiefel zur Korrektur eines Sichelfußes. Dieser besteht wiederum aus zwei schalenförmigen Fassungen für Ferse und Vorderfuß, die im Sohlenbereich voneinander getrennt sind und hier flache winkelförmige Beschläge aufweisen, die durch ein mediales Gelenk und eine laterale Zugfeder miteinander verbunden sind. Hierbei ergeben sich ähnliche Nachteile wie beim vorerwähnten Stand der Technik.

Die DE-C-215 555 zeigt eine Korrekturvorrichtung für Platt- und Klumpfüße mit einem auf der Lateralseite des Fußes angeordneten federnden Metallbogen, der durch einen über Vorderfuß und Ferse geschlungenen Riemen am Fuß befestigt wird. Durch eine Stellschraube wird der Metallbogen in seiner Weite verändert und über die Riemen dementsprechend stärker oder schwächer gegen den Fuß gepreßt. Hierbei besteht ein direkter Berührungskontakt zwischen Metallbogen und Fuß auf der Lateralseite. Der Metallbogen kann in der Mitte auch ein vertikales Drehgelenk haben. Diese Anordnung läßt sich durch die unsichere Riemenführung nicht zuverlässig einstellen. Sie erlaubt ebenfalls weder ein Gehen noch ein Stehen des Patienten.

Aus der DE-C-95 326 ist ein anderer Apparat für die Behandlung des Platt- oder Klumpfußes bekannt, bei dem die beiden Fußfassungen durch ein seitliches starres Beschlagteil mit einem Gelenk nebst Klinkensperre verbunden sind. Das Gelenk hat eine horizontale Drehachse und dient zur Korrektur von Fehlstellungen des Fußes in der Vertikalen.

Die DE-C-302 366 lehrt eine Vorrichtung zur Verhinderung des Herabhängens gelähmter Füße, bei der die beiden Fußfassungen durch gekreuzte Zugbänder miteinander verbunden sind, die von der Wade ausgehend schräg abwärts zum Vorderfuß verlaufen und eine nach oben gerichtete Zugkraft für den herabhängenden Fuß ausüben sollen.

Eine kombinierte Klumpfuß- und Sichelfußschiene ist aus dem DE-U-76 35 626 bekannt. Zur Sichelfußkorrektur ist ein sohlenseitiger Verbindungsbeschlag zwischen den Fußfassungen vorhanden, der ein Rastengelenk mit vertikaler Drehachse aufweist. Eine solche Beschlaganordnung zeigt auch das DE-U-94 18 854. In beiden Fällen kann der Patient weder stehen noch gehen.

Die DE-A-43 18 523 offenbart ein Stützgerät zum Geradrichten einer Valgußgroßen-Zehe, die ein zwischen die Zehen einzuführendes und durch Stützbänder gehaltenes elastisches Formkissen besitzt. Aus der DE-C-359 070 ist eine Plattfußeinlage bekannt, die als einteilige Korrektursohle mit t-förmigen Gummibändern im Wölbungsbereich versehen ist.

Es ist daher Aufgabe der vorliegenden Erfindung, eine bessere Sichelfuß-Korrekturschiene aufzuzeigen.

Die Erfindung löst diese Aufgabe mit den Merkmalen im Hauptanspruch.
Bei der erfindungsgemäßen Sichelfuß-Korrekturschiene erfolgt die Korrektur der Fehlstellung des Fußes durch ein seitliches bandförmiges Stellelement, das die Vorderfußfassung und die Fersenfassung miteinander verbindet und eine Zugkraft ausübt. Das Band erlaubt eine feinfühlige und genaue Korrektur der Fehlstellung. Es kann gegebenenfalls auch etwas nachgeben. Das Band hat den Vorteil eines günstigen Kraftangriffes und einer verbesserten Stellwirkung für die Fußfassungen.

Das zwischen den Fußfassungen angeordnete seitliche Verbindungsteil bildet das Gelenk zwischen Vorderfuß- und Fersenfassung und ist hierzu entsprechend biegeelastisch ausgebildet. Dies hat den Vorteil einer besonders einfachen und kostengünstigen Ausgestaltung. Das Verbindungsteil kann gegebenenfalls auch mit einer Wandschwächung zur genaueren Festlegung der Dreh- oder Biegeachse ausgestattet sein.

Das Band ist für die Korrektur der klassischen nach medial gerichteten Fehlstellung lateral angeordnet und übt einen permanenten und gegebenenfalls federnden Zug auf die Vorderfußfassung aus. Der Vorderfuß wird stets nach lateral gezogen und dadurch die Fehlstellung korrigiert.

Die Bänder können als elastisches und vorzugsweise gummiförmiges Band oder als dehnungsarmes Spannband ausgebildet sein. Das Spannband erlaubt eine feste und kaum nachgiebige Einstellung der Zugkraft. Beim elastischen Band ist in Grenzen ein Nachgeben möglich. Dadurch kann die Sichelfuß-Korrekturschiene auf unterschiedliche Korrektur-Erfordernisse eingestellt werden. Die Bänder lassen sich ohne Änderung der anderen Schienenkomponenten austauschen.

Die Bänder sind verstellbar an den Fußfassungen angeordnet, wobei die Verstellung vorzugsweise über einen Klettverschluß vorgenommen wird. Dies ermöglicht eine einfache und genaue Korrektureinstellung. Diese kann gegebenenfalls auch vom Patienten bei einer Unverträglichkeit selbst verändert und nachgestellt werden. Der Klettverschluß erlaubt gegebenenfalls auch ein Öffnen und Abnehmen der Sichelfuß-Korrekturschiene.

Die Bänder und das Gelenk mit dem Verbindungsteil sind seitlich am Fuß angeordnet. Dadurch befinden sich im Sohlenbereich keine störenden Teile. Außerdem ist die Kraftwirkung und die Gelenkwirkung besser als bei Sohlenbeschlägen. Eine besonders wirksame Korrektureinstellung ergibt sich, wenn das Gelenk medial und das Band lateral angeordnet ist. Hierdurch ist auch der Verstellwinkel größer als bei einer lateralen Gelenkanordnung. Zur Abstützung der Korrektur- und Spannkräfte am Fuß und zur Verhinderung von Druckstellen oder von Blasenbildung sind in den Fußfassungen an geeigneten Stellen Pelotten und gegebenenfalls ein die Fußwölbung unterstützendes Längsgewölbe angeordnet.

In den Unteransprüchen sind weitere vorteilhafte Ausgestaltungen der Erfindung angegeben.

Die Erfindung ist in den Zeichnungen beispielsweise und schematisch dargestellt. Im einzelnen zeigen
- Figur 1:: eine Sichelfuß-Korrekturschiene mit einem lateralen Gelenk und einem Stellelement in Form eines elastischen Bandes, die nicht unter Anspruch 1 fällt und die im Dokument DE 298 12 781 U1 am 10.09.1998 veröffenthicht wurde, und
- Figur 2:: eine Variante zu Figur 1 mit einem medialen Gelenk und einem Stellelement in Gestalt eines Spannbandes.

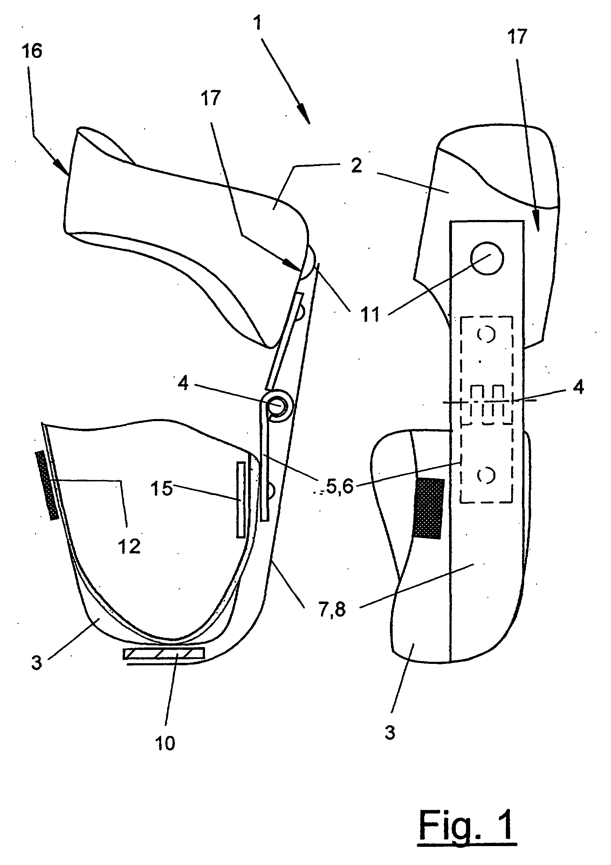

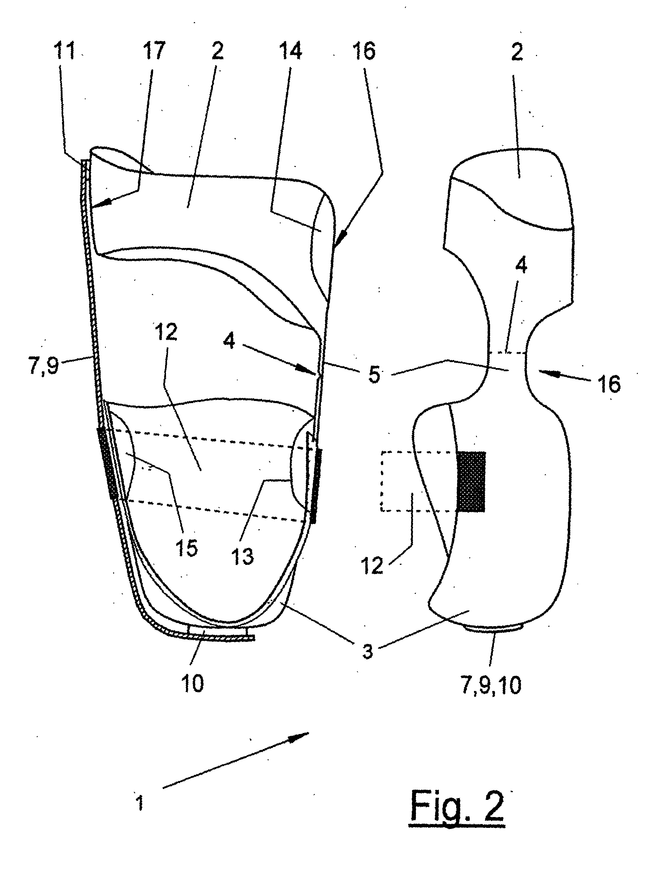

In den Zeichnungen von Figur 1 und 2 sind zwei Sichelfuß-Korrekturschienen (1) jeweils in Draufsicht und geklappter Seitenansicht dargestellt. In beiden Fällen besteht die Sichelfuß-Korrekturschiene (1) aus einer Vorderfußfassung (2) und einer Fersenfassung (3), die an die Fußform angepaßt sind. Sie sind gegebenenfalls als offene Schalen ausgebildet und mit einem obenliegenden Klettband (12) oder einem anderen Befestigungselement zur Fixierung des Fußes (nicht dargestellt) versehen. Die Vorderfußfassung (2) kann auch ringförmig gestaltet sein.

Die beiden Fassungen (2,3) sind vorzugsweise mit Distanz voneinander angeordnet und durch ein gemeinsames Verbindungsteil (5) mit einem Gelenk (4) aneinander gekoppelt. Dem Gelenk (4) ist ein bandförmiges Stellelement (7) für die Einrichtung der Winkelstellung der Fassungen (2,3) zugeordnet.

Das Gelenk (4), das Verbindungsteil (5) und das Stellelement (7) sind in beiden Fällen an der Seite des Fußes und mit etwas Abstand über der Sohle angeordnet. In beiden Sichelfuß-Korrekturschiene befindet sich das Gelenk (4) etwa in der Mitte zwischen den beiden distanzierten Fassungen (2,3). Alternativ kann das Gelenk auch weiter vorn oder hinten sitzen.

In Figur 1 sind dabei das Gelenk (4) und das Verbindungsteil (5) auf der lateralen Fußseite (17) zu finden. Figur 2 zeigt eine Variante mit einer Anordnung auf der medialen Fußseite (16). Das bandförmige Stellelement (7,8,9) befindet sich in beiden Fällen auf der lateralen Fußseite (17) und übt eine nach lateral gerichteten Zugkraft auf die Vorderfußfassung (2) bzw. den Vorderfuß aus.

In Figur 1 besteht das Stellelement (7) aus einem elastischen Band (8), z.B. einem Gummiband, das die Vorderfußfassung (2) gegenüber der Fersenfassung (3) federnd nach lateral zieht und dementsprechend das Gelenk (4) zu drehen versucht. In dieser aus DE 298 12 781 U1 bekannten Sichelfuß-Korrekturschiene erstreckt sich das elastische Band (8) im wesentlichen horizontal und übergreift außenseitig das Gelenk (4) und das Verbindungsteil (5).

In der Variante von Figur 2 ist das bandförmige Stellelement (7) als Spannband (9) ausgebildet, das eine große Dehnungsfestigkeit besitzt und auf Zug nicht oder nur wenig nachgibt. Das Spannband (9) kann aus einem Gewebeband oder einem beliebigen anderen biegeelastischen und dabei dehnungsfesten Werkstoff bestehen.

Das Spannband (9) befindet sich auch in der erfindungsgemässen Schiene von Figur 2 auf der lateralen Fußseite (17). Das Gelenk (4) kann bei dieser Variante auf der medialen Fußseite (16) angeordnet sein. Alternativ ist auch eine laterale Anordnung ähnlich Figur 1 möglich. Bei der in Figur 2 gezeigten medialen Gelenkanordnung greift das Spannband (9) durch seinen seitlichen Abstand mit einem vergrößerten Hebelarm gegenüber dem Gelenk (4) an. Diese Gelenkanordnung bietet auch größere Verstellwinkel als die Variante von Figur 1.

Das Verbindungsteil (5) und das Gelenk (4) sind in Figur 2 ebenfalls anders als in Figur 1 ausgebildet. Das Verbindungsteil (5) besteht hier aus einem durchgehenden und die beiden Fußfassungen (2,3) verbindenden seitlichen Fortsatz. Diese Gestaltung erlaubt eine einteilige Ausführung der Fußfassungen (2,3) und des Verbindungsteiles (5). Diese Teile können z.B. aus einem thermoplastischen Kunststoff bestehen und lassen sich gegebenenfalls als Spritzteil miteinander herstellen.

Das Gelenk (4) wird in Figur 2 durch eine biegeelastische Ausbildung des Verbindungsteiles (5) geschaffen. Hierzu kann das Verbindungsteil (5) eine Wandschwächung besitzen. Zum einen kann es relativ dünnwandig ausgebildet sein, wodurch sich schon von allein eine Biegeelastizität um die vertikale Achse ergibt. Außerdem kann das Verbindungsteil zusätzlich eine Einkerbung oder eine andere spezielle Wandschwächung besitzen, die in der gewünschten Drehachse, vorzugsweise vertikal, verläuft und diese Drehachse näher definiert. Diese Einkerbung kann sich z.B. in der Mitte des Verbindungsteiles (5) befinden. Außerdem kann das Verbindungsteil (5) oben und unten eine Einschnürung gemäß der Seitendarstellung von Figur 2 aufweisen. Diese schmale Ausbildung erleichtert ebenfalls die Biegelastizität.

Alternativ sind noch weitere Varianten möglich.

Die Bänder (8,9) sind vorzugsweise an der Vorderfußfassung (2) durch einen Niet (11) oder dergleichen starr befestigt. An der Fersenfassung (3) sind sie durch einen Klettverschluß (10) etwa in Fersenmitte verstellbar befestigt. Alternativ kann ein Klettverschluß (10) auch an der Vorderfußfassung (2) angeordnet sein.

Gemäß Figur 2 können in den Fußfassungen (2,3) seitliche Pelotten (14,15) zur weichen Anlage am Fuß vorhanden sein. Die Pelotte (14) an der Vorderfußfassung (2) ist vorzugsweise dem Stellelement (7) gegenüberliegend angeordnet, das heißt in der Variante von Figur 2 medial. An der Fersenfassung (3) ist die Pelotte (15) auf der Seite des Stellelementes (7) und damit lateral angeordnet. Die Fußfassungen (2,3), insbesondere die Fersenfassung (3), kann zudem noch ein die Fußwölbung unterstützendes Längsgewölbe (13) auf der medialen Fußseite (16) aufweisen.

Abwandlungen des gezeigten Ausführungsbeispiels sind in verschiedener Weise möglich. Die beiden Fußfassungen (2,3) können abweichend von Figur 1 und 2 näher zusammenrücken, wodurch sich auch das Verbindungsteil (5) verkürzt. In dem gezeigten Ausführungsbeispiel ist der Abstand der Fußfassungen (2,3) vorgegeben und richtet sich nach den genormten Fußgrößen. Alternativ kann das Verbindungsteil (5) eine geeignete Längsverstellung zur Veränderung dieses Abstands besitzen. Eine weitere Verstellbarkeit ist auch hinsichtlich der Pelotten (14,15) und des Längsgewölbes (13) vorgesehen, die z.B. über Klettverschlüsse oder dergleichen andere lösbare Verbindungen an den Fußfassungen (2,3) befestigt sind. Das elastische Band (8) kann einen zugfesten Bandabschnitt mit einem zwischengeschalteten federnden Zugelement besitzen. Darüberhinaus sind weitere Abwandlungen möglich.

In einer nicht dargestellten Variante können die Bänder (8,9) auch medial (16) angeordnet sein und eine nach medial gerichtete Zug- oder Stellkraft auf den Fuß ausüben.

### BEZUGSZEICHENLISTE

- 1: Sichelfuß-Korrekturschiene
- 2: Vorderfußfassung
- 3: Fersenfassung
- 4: Gelenk
- 5: Verbindungsteil
- 6: Beschlag, Bügel
- 7: Stellelement
- 8: elastisches Band
- 9: Spannband
- 10: Klettverschluß
- 11: Niet
- 12: Klettband
- 13: Längsgewölbe
- 14: Pelotte, Vorderfuß
- 15: Pelotte, Ferse
- 16: mediale Seite
- 17: laterale Seite

## Patentansprüche

1. Sichelfuß-Korrekturschiene mit einer Fersenfassung (3) und einer Vorderfußfassung (2), die in Distanz zueinander angeordnet und durch ein seitliches Verbindungsteil (5) sowie ein Gelenk (4) miteinander verbunden und mit einem Stellelement (7) für die korrigierende Winkelstellung der Fassungen (2,3) ausgerüstet sind, wobei das Stellelement (7) als Band ausgebildet ist, das an den Fußfassungen (2,3) verstellbar befestigt ist und wobei das Verbindungsteil (5) zumindest stellenweise biegeelastisch ausgebildet ist und das Gelenk (4) bildet.

2. Sichelfuß-Korrekturschiene nach Anspruch 1, **dadurch gekennzeichnet, dass** Verbindungsteil (5) dünnwandig ausgebildet ist.

3. Sichelfuß-Korrekturschiene nach Anspruch 1 oder 2, **dadurch gekennzeichnet, dass** Verbindungsteil (5) eine Wandschwächung, insbesondere eine Einkerbung oder eine Einschnürung besitzt.

4. Sichelfuß-Korrekturschiene nach Anspruch 1, 2 oder 3, **dadurch gekennzeichnet, dass** Verbindungsteil (5) als seitlich durchgehender Verbindungsfortsatz der Fußfassungen (2,3) ausgebildet ist.

5. Sichelfuß-Korrekturschiene nach einem der Ansprüche 1 bis 4, **dadurch gekennzeichnet, dass** das Verbindungsteil (5) mit dem Gelenk (4) an der einen, vorzugsweise der medialen Fußseite (16) und das Stellelement (7) auf der gegenüberliegenden, vorzugsweise der lateralen Fußseite (17) angeordnet sind.

6. Sichelfuß-Korrekturschiene nach Anspruch 5, **dadurch gekennzeichnet, dass** das Stellelement (7) als biegeelastisches und dehnungsfestes Spannband (9) ausgebildet ist.

7. Sichelfuß-Korrekturschiene nach einem der Ansprüche 1 bis 4, **dadurch gekennzeichnet, dass** das Verbindungsteil (5) mit dem Gelenk (4) und das Stellelement (7) auf der gleichen, vorzugsweise der lateralen Fußseite (17) angeordnet sind.

8. Sichelfuß-Korrekturschiene nach Anspruch 7, **dadurch gekennzeichnet, dass** das Stellelement (7) als elastisches Band ausgebildet ist.

9. Sichelfuß-Korrekturschiene nach einem der Ansprüche 1 bis 8, **dadurch gekennzeichnet, dass** das Band zumindest an einer Fassung (2,3) mit einem Klettverschluß (10) verstellbar befestigt ist.

10. Sichelfuß-Korrekturschiene nach einem der Ansprüche 1 bis 9, **dadurch gekennzeichnet, dass** die Fußfassungen (2,3) Pelotten (14,15) und ein Längsgewölbe (13) aufweisen.

11. Sichelfuß-Korrekturschiene nach einem der Ansprüche 1 bis 10, **dadurch gekennzeichnet, dass** die Fußfassungen (2,3) zumindest teilweise als offene Schalen ausgebildet und mit ein oder mehreren Klettbändern (12) zur Fixierung des Fußes ausgerüstet sind.

12. Sichelfuß-Korrekturschiene nach einem der Ansprüche 1 bis 11, **dadurch gekennzeichnet, dass** die Fußfassungen (2,3) und das Verbindungsteil (5) aus einem thermoplastischen Kunststoff bestehen.

## Claims

1. Corrective rail for talipes varus having a heel holder (3) and a forefoot holder (2) which are arranged at a distance from each other, are connected to each other by a lateral connecting part (5) and a joint (4) and are equipped with an adjusting element (7) for the corrective angular position of the holders (2, 3), the adjusting element (7) being designed as a strap, which is fastened adjustably to the foot holders (2, 3), and the connecting part (5) being of flexurally elastic design at least at some points and forming the joint (4).

2. Corrective rail for talipes varus according to Claim 1, **characterized in that** the connecting part (5) is of thin-walled design.

3. Corrective rail for talipes varus according to Claim 1 or 2, **characterized in that** the connecting part (5) has a weakened wall area, in particular a notch or a constriction.

4. Corrective rail for talipes varus according to Claim 1, 2 or 3, **characterized in that** the connecting part (5) is designed as a laterally continuous connecting extension of the foot holders (2, 3).

5. Corrective rail for talipes varus according to one of Claims 1 to 4, **characterized in that** the connecting part (5) is arranged together with the joint (4) on the one foot side (16), preferably the medial foot side, and the adjusting element (7) is arranged on the opposite foot side (17), preferably the lateral foot side.

6. Corrective rail for talipes varus according to Claim 5, **characterized in that** the adjusting element (7) is designed as a flexurally elastic and extension-proof tightening strap.

7. Corrective rail for talipes varus according to one of Claims 1 to 4, **characterized in that** the connecting part (5) together with the joint (4), and the adjusting element (7) are arranged on the same foot side (17), preferably the lateral foot side.

8. Corrective rail for talipes varus according to Claim 7, **characterized in that** the adjusting element (7) is designed as an elastic strap.

9. Corrective rail for talipes varus according to one of Claims 1 to 8, **characterized in that** the strap is fastened adjustably at least to one holder (2, 3) with a touch-and-close fastener (10).

10. Corrective rail for talipes varus according to one of Claims 1 to 9, **characterized in that** the foot holders (2, 3) have cushioning pads (14, 15) and a longitudinal arch (13).

11. Corrective rail for talipes varus according to one of Claims 1 to 10, **characterized in that** the foot holders (2, 3) are designed at least in part as open shells and are equipped with one or more touch-and-close straps (12) for fixing the foot in place.

12. Corrective rail for talipes varus according to one of Claims 1 to 11, **characterized in that** the foot holders (2, 3) and the connecting part (5) consist of a thermoplastic.

## Revendications

1. Attelle de correction pour pied falciforme comportant une monture de talon (3) et une monture de pied antérieure (2), qui sont disposées à distance l'une de l'autre, reliées par une pièce de liaison latérale (5) ainsi que par une articulation (4) et équipées d'un élément (7) de réglage de la position angulaire corrective des montures (2, 3), l'élément de réglage (7) étant conformé en bande qui est fixée de façon réglable aux montures de pied (2, 3) et la pièce de liaison (5) étant conformée de façon à être au moins partiellement élastique en flexion et formant l'articulation (4).

2. Attelle de correction pour pied falciforme selon la revendication 1, **caractérisée en ce que** la pièce de liaison (5) a des parois minces.

3. Attelle de correction pour pied falciforme selon la revendication 1 ou 2, **caractérisée en ce que** la pièce de liaison (5) possède un élément de paroi de moindre résistance mécanique, en particulier une rainure ou une partie rétrécie.

4. Attelle de correction pour pied falciforme selon la revendication 1, 2 ou 3, **caractérisée en ce que** la pièce de liaison (5) est conformée en prolongement de liaison, s'étendant latéralement d'un seul tenant, des montures de pied (2, 3).

5. Attelle de correction pour pied falciforme selon l'une des revendications 1 à 4, **caractérisée en ce que** la pièce de liaison (5) dotée de l'articulation (4) est disposée sur l'un des côtés du pied, de préférence le côté médian (16), et l'élément de réglage (7) est disposé sur le côté opposé du pied, de préférence le côté latéral (17).

6. Attelle de correction pour pied falciforme selon la revendication 5,
**caractérisée en ce que** l'élément de réglage (7) est conformé en bande de serrage élastique en flexion et rigide en extension.

7. Attelle de correction pour pied falciforme selon l'une des revendications 1 à 4, **caractérisée en ce que** la pièce de liaison (5) dotée de l'articulation (4) et l'élément de réglage (7) sont disposés sur même côté du pied, de préférence le côté latéral (17).

8. Attelle de correction pour pied falciforme selon la revendication 7, **caractérisée en ce que** l'élément de réglage (7) est conformé en bande élastique.

9. Attelle de correction pour pied falciforme selon l'une des revendications 1 à 8, **caractérisée en ce que** la bande est fixée de façon réglable au moins à une monture (2, 3) au moyen d'une fermeture (10) autoagrippante à boucles et crochets.

10. Attelle de correction pour pied falciforme selon l'une des revendications 1 à 9, **caractérisée en ce que** les montures de pied (2, 3) comportent des bourrelets (14, 15) et une cambrure longitudinale (13).

11. Attelle de correction pour pied falciforme selon l'une des revendications 1 à 10, **caractérisée en ce que** les montures de pied (2, 3) sont conformées au moins partiellement en coques ouvertes et sont équipées d'une ou plusieurs bandes autoagrippantes à boucles et crochets (12) pour la fixation du pied.

12. Attelle de correction pour pied falciforme selon l'une des revendications 1 à 4, **caractérisée en ce que** les montures de pied (2, 3) et la pièce de liaison (5) sont matière thermoplastique.
